(19) Europäisches Patentamt
European Patent Office
Office européen des brevets

(11) Publication number: **0 025 287 A1**

# (12) EUROPEAN PATENT APPLICATION

(21) Application number: 80302837.2

(22) Date of filing: 18.08.80

(51) Int. Cl.³: C 07 D 498/04
A 61 K 31/42
//(C07D498/04, 205/00, 263/00)

(30) Priority: 23.08.79 GB 7929436
23.08.79 GB 7929435
23.08.79 GB 7929437
21.08.79 GB 7929139
21.08.79 GB 7929132
21.08.79 GB 7929105

(43) Date of publication of application:
18.03.81 Bulletin 81/11

(84) Designated Contracting States:
AT BE CH DE FR GB IT LI LU NL SE

(71) Applicant: BEECHAM GROUP LIMITED
Beecham House Great West Road
Brentford, Middlesex(GB)

(72) Inventor: Denerley, Paul Millington
45 Avondale Close
Horley Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

## (54) Clavulanic acid derivatives, their preparation, pharmaceutical compositions containing them and their preparation.

(57) This invention provides compounds of the formula (I):

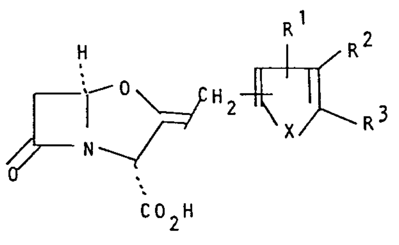

(1)

and pharmaceutically acceptable salts and in-vivo hydrolysable esters thereof wherein $R^1$ is a hydrogen atom or a $C_{1-6}$ alkyl group, $R^2$ is a hydrogen atom or a $C_{1-6}$ alkyl group, $R^3$ is a hydrogen atom or a $C_{1-6}$ alkyl group, or $R^2$ and $R^3$ together with the carbon atoms to which they are attached form a benzene ring which optionally is substituted by a $C_{1-6}$ alkyl, $C_{1-6}$ alkoxy or hydroxy group; and X is a sulphur or oxygen atom or is a group $NR^4$ wherein is a hydrogen atom or a $C_{1-6}$ alkyl group, which compounds have β-lactamase inhibitory and antibacterial properties. A process for their preparation is described, as is their use in pharmaceutical compositions.

EP 0 025 287 A1

TITLE MODIFIED
see front page

CLAVULANIC ACID DERIVATIVES, THEIR PREPARATION AND USE

This invention relates to a novel series of 9-heterocyclyl-deoxyclavulanic acid derivatives, to a method for their preparation, to their use as antibacterial and β-lactamase inhibitory agents and to pharmaceutical compositions comprising such compounds.

British Patent Specification Number 1508977 discloses clavulanic acid and pharmaceutically acceptable salts and esters thereof. It has now been discovered that derivatives of clavulanic acid may be prepared that have a heterocyclyl group at the 9-position in place of the hydroxyl group.

Thus the present invention provides a compound of the formula (I):

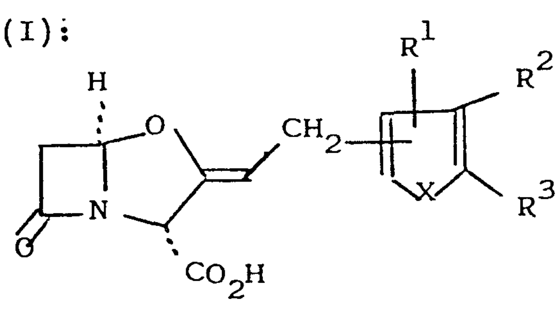

(I)

and pharmaceutically acceptable salts and in-vivo hydrolysable esters thereof wherein $R^1$ is a hydrogen atom or a $C_{1-6}$ alkyl group, $R^2$ is a hydrogen atom or a $C_{1-6}$ alkyl group, $R^3$ is a hydrogen atom or a $C_{1-6}$ alkyl group, or $R^2$ and $R^3$ together with the carbon atoms to which they are attached form a benzene ring which optionally is substituted by a $C_{1-6}$ alkyl, $C_{1-6}$ alkoxy or hydroxy group; and X is a sulphur or oxygen atom or is a group $NR^4$ wherein $R^4$ is a hydrogen atom or a $C_{1-6}$ alkyl group.

Suitable values for $R^1$, $R^2$ and $R^3$ when independent include those selected from the group consisting of hydrogen, methyl, ethyl, propyl and butyl. Of these the hydrogen atom and the methyl group are preferred.

Suitably $R^2$ and $R^3$ together with the carbon atoms to which they are attached form a benzene ring which is optionally substituted by $C_{1-6}$ alkyl, $C_{1-6}$ alkoxy or a hydroxy group. When such a benzene ring is formed suitably it is not substituted, or it is substituted by a group selected from methoxy, ethoxy, methyl, ethyl and hydroxy.

Suitably X is a sulphur atom thus forming either a thiophene or thionaphthene ring system.

Alternatively X is an oxygen atom thus forming either a furan or benzofuran ring system.

In a further aspect X is a $-NR^4-$ group thus forming a pyrrole or indole ring system. Preferably $R^4$ is a hydrogen atom or a methyl group.

A favoured sub-group of compounds is that of the formula (II):

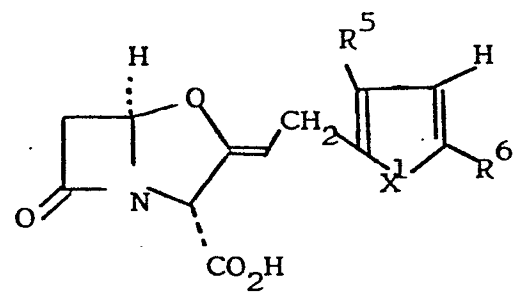

(II)

and pharmaceutically acceptable salts and in-vivo hydrolysable esters thereof wherein $X^1$ is an oxygen or sulphur atom or a -NH- or $-N(CH_3)-$ group; $R^5$ is a hydrogen atom or a methyl group and $R^6$ is a hydrogen atom or a methyl group. Preferably one of $R^5$ and $R^6$ is a hydrogen atom and the other is a methyl group. Alternatively $R^5$ and $R^6$ are independently both hydrogen atoms.

Examples of the compounds of this invention include:

9-pyrr-2-yldeoxyclavulanic acid,
9-(1-methyl)pyrr-2-yldeoxyclavulanic acid,
9-(3-methyl)thien-2-yldeoxyclavulanic acid,
9-(5-methyl)fur-2-yldeoxyclavulanic acid,
9-(3-methyl)benzofur-2-yldeoxyclavulanic acid,
9-indol-3-yldeoxyclavulanic acid,
9-thionaphthyldeoxyclavulanic acid,

and pharmaceutically acceptable salts thereof.

Suitably the compounds of the formulae (I)-(II) are presented as the free acid but more favourably they are presented in the form of a salt. Suitable salts include the alkali and alkaline earth metal salts such as the sodium, magnesium, potassium or calcium salts. Other suitable salts include the ammonium salt and substituted ammonium salts such as the methylamine, ethylamine, dimethylamine, tertiary-butylamine and tetramethylammonium salts.

Particularly suitable salts include the sodium, potassium, calcium and magnesium salts; of these the sodium and potassium are preferred.

The compounds of the formulae (I)-(II) may be provided as in-vivo hydrolysable esters. Such esters are those which hydrolyse in the human body to produce the parent acid. Suitable in-vivo hydrolysable esters include those esters known to give in-vivo hydrolysis in penicillins. Thus suitable esters include those of the formula (i):

$$-CO-O-CHR^7-O-CO-R^8 \qquad (i)$$

wherein $R^7$ is a hydrogen atom, or a methyl or phenyl group; $R^8$ is an alkyl group of 1 to 6 carbon atoms, a phenyl group, an alkyl group of 1 to 3 carbon atoms substituted by a phenyl group, an alkoxy group of 1 to 6 carbon atoms, a phenoxy group, or an alkoxy group of 1 to 3 carbon atoms substituted by a phenyl group; or $R^7$ is attached to $R^8$ to form a 1,2-diphenylene or

4,5-dimethoxy-1,2-diphenylene group.

Favourably $R^7$ is hydrogen.

When $R^7$ is hydrogen suitably $R^8$ is selected from methyl, ethyl, n-propyl, iso-propyl, n-butyl, tert-butyl, phenyl, benzyl, methoxy, ethoxy, n-propyloxy and iso-propyloxy. Preferably $R^8$ is tert-butyl.

Favourably $R^7$ and $R^8$ are joined so that the ester is a phthalidyl or 3,4-dimethoxyphthalidyl ester.

Of these esters those favoured as in-vivo hydrolysable esters are the acetoxymethyl, acetoxyethyl, phthalidyl, ethoxycarbonyloxymethyl, α-ethoxycarbonyloxy-ethyl and pivaloyloxymethyl esters.

The in-vivo hydrolysable nature of the ester may be confirmed by administration to an animal such as a mouse or rat and determination of the presence of a compound of the formula (I) or a salt thereof in the blood or urine of the animal. Alternatively hydrolysis in human blood or serum may be determined.

In another aspect the present invention provides pharmaceutical compositions which comprise a compound of formula (I) or a pharmaceutically acceptable salt or

a pharmaceutically acceptable salt or in-vivo hydrolysable ester thereof and a pharmaceutically acceptable carrier.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives and disintegrants in accordance with conventional pharmaceutical practice in the manner well understood by those skilled in the art of formulating antibiotics.

Injectable or infusable compositions of salts of a compound of the formula (I) are particularly suitable as high tissue levels of a compound of the formula (I) can occur after administration by injection or infusion. Thus, one preferred composition aspect of this invention comprises a salt of a compound of the formula (I) in sterile form.

Unit dose compostions comprising a compound of the formula (I) or a salt or ester thereof adapted for oral administration form a further preferred aspect of this invention.

The compound of the formula (I) or its salt or ester may be present in the composition as sole therapeutic agent or it may be present together with other therapeutic

agents such as a penicillin or cephalosporin. Suitable penicillins or cephalosporins for inclusion in the compositions of this invention include benzylpenicillin, phenoxymethylpenicillin, carbenicillin, azidocillin, propicillin, ampicillin, epicillin, ticarcillin, cyclacillin, cefatriazine, pirbenicillin, α-sulphonyloxy-benzylpenicillin, cephaloridine, cephalothin, cefazolin, cephalexin, cephacetrile, cephamandole nafate, cephapirin, cephradine, 4-hydroxycephalexin, cefaparole, cephaloglycine, and other well known penicillins and cephalosporins or pro-drugs therefor such as hetacillin, metampicillin, 4-acetoxyampicillin, the acetoxymethyl, ethoxycarbonyloxymethyl, pivaloyloxymethyl or phthalidyl esters of benzylpenicillin, ampicillin, amoxycillin or cephaloglycine, or the phenyl, tolyl or indanyl α-esters of carbenicillin or ticarcillin. Such compounds are frequently used in the form of a salt or hydrate.

Naturally if the penicillin or cephalosporin present in the composition is not suitable for oral administration then the composition will be adapted for parenteral administration.

When present in a pharmaceutical composition together with a penicillin or cephalosporin, the ratio of a compound of the formula (I) or its salt or ester present to penicillin or cephalosporin present may vary over a wide range of ratios, for example 3:1 to 1:10 and advantageously may be from 2:1 to 1:5 for example, 1:1 to 1:3. The total quantity of antibacterial

agents present in any unit dosage form will normally be between 50 and 1500 mg and will usually be between 100 and 1000 mg.

Compositions of this invention may be used for the treatment of infections on *inter alia*, the respiratory tract, the urinary tract and soft tissues and mastitis in cattle.

Normally between 50 and 3000mg of the compound of the invention will be administered each day of treatment but more usually between 100 and 1000 mg of the compounds of the invention will be administered per day, for example as 1-6 doses, more usually 2-4 doses.

The penicillin or cephalosporin in synergistic compositions of this invention will normally be present up to or at approximately the amount at which it is conventionally used.

Particularly favoured compositions of this invention will contain from 150-1000 mg of amoxycillin, ampicillin or a pro-drug therefor and from 50-500 mg of a compound of the formula (I) or a salt or *in-vivo* hydrolysable ester thereof and more suitably from 200-500 mg of amoxycillin, ampicillin or a pro-drug therefor and from 50-250 mg of a compound of the formula (I) or a salt or *in-vivo* hydrolysable ester thereof.

The materials present in such compositions may be hydrated if desired, for example ampicillin trihydrate or amoxycillin trihydrate may be employed. The weights of the antibiotics in such compositions are expressed on the basis of antibiotic theoretically available from the composition and not on the basis of the weight of pro-drug.

The compounds of the formula (I) may be prepared by the arylation of an ester of clavulanic acid. Thus in a further aspect the present invention provides a process for the preparation of a compound of the formula (I) or a pharmaceutically acceptable salt or in-vivo hydrolysable ester thereof which process comprises the reaction of an ester of clavulanic acid of the formula (III):

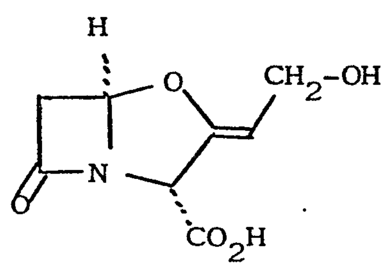

(III)

with a compound of the formula (IV):

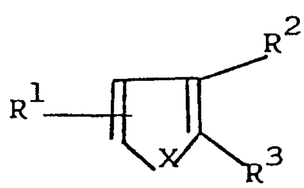

(IV)

wherein X, $R^1$, $R^2$ and $R^3$ are as defined in relation to formula (I); in the presence of a catalyst; and thereafter:

i) converting any ester that is not in-vivo hydrolysable into a free acid or a pharmaceutically acceptable salt or in-vivo hydrolysable ester;

ii) optionally converting any in-vivo hydrolysable ester into a free acid, a pharmaceutically acceptable salt or different in-vivo hydrolysable ester.

By the term a catalyst used herein we mean an entity that catalyses the alkylation reaction of an aromatic ring by an alcohol. Preferably the catalyst employed is one which is knownto catalyse reactions known as Friedel - Crafts reactions. Suitable catalysts include aluminium trichloride, zinc chloride, zinc bromide, antimony pentachloride, ferric choride, stannic chloride, boron trifluoride etherate and chemical equivalents thereof. Of these boron trifluoride is preferred, for example as the etherate.

The position at which the 9-deoxyclavulanic acid moiety becomes attached to the heterocycle is influenced by the nature of the heterocycle and also by the nature of the substituents $R^1$, $R^2$ and $R^3$. In general if the compound of the formula (IV) is monocyclic the 9-deoxyclavulanate moiety will attach to the carbon atom adjacent to the heteroatom (i.e. the 2- or the 5- position in formula (V)):

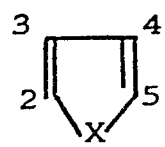

(V)

if the 2- and the 5- positions are blocked then the point of attachment will be the 3- or 4- position. If the compound of the formula (IV) is an indole or thionaphthene then the likely point of attachment for the 9-deoxyclavulanate moiety is the 3- position (see formula (VI)):

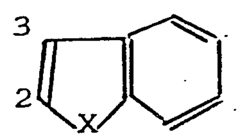

(VI)

unless of course this is blocked in which case the 2-position is favoured. For the benzofuran ring system it is likely that the point of attachment of the 9-deoxyclavulanate moiety is the 2- position unless of course this is blocked in which case the 3- position is favoured.

The reaction is generally carried out in the presence of a solvent, though the identity of the particular solvent selected is not critical to the success of the reaction provided that it is able to form a homogeneous solution of the compound of the formula (IV) and the ester of clavulanic acid and is substantially inert to the starting materials, product and catalyst.

Suitable solvents may be selected by trial and error. We have found generally that the reaction may be conveniently carried out in non-polar organic solvents such as halogenated hydrocarbons e.g. chloroform, carbon tetrachloride, methylene chloride and ethylene chloride.

The temperature at which the reaction is carried out depends upon the particular ester and heterocycle which have been selected and upon the nature of the catalyst.

Generally, the reaction is carried out at moderate temperatures to low temperatures, i.e. less than 50°C and as low as -70°C. In order to prevent side reactions, it is generally convenient to cool the reagents to a temperature of 0°C or below prior to mixing, to mix the reagents while cold and thereafter maintain the reaction mixture at a temperature between 0°C and -30°C.

It is of course understood that best results are obtained when reactions of the type described here are carried out under an atmosphere of dry air or a dry inert gas such as argon or nitrogen.

The ester of the compound (I) produced may be isolated from the reaction mixture by any standard method such as fractional crystallisation, countercurrent separation or chromatography. We have found

that it is most convenient to separate the desired product by column chromatography.

Any convenient ester of clavulanic acid may be used in the process of this invention. Since it is frequently desirable to form a salt of compounds (I), the ester of clavulanic acid employed is preferably one which is readily converted to the parent acid or its salt by mild methods of hydrolysis or hydrogenolysis. For example see the disclosures of British Patent Specification Numbers 1508977 and 1508978. Particularly suitable esters of clavulanic acid for use in this process include the methoxymethyl or methyl esters, or an optionally substituted benzyl ester, optionally substituted in the para position by a lower alkyl, lower alkoxy or nitro group or by a halogen atom.

A preferred ester for use in this process is benzyl clavulanate.

Suitable methods for hydrolysing esters of compounds (I) include mild base hydrolysis in aqueous solution. The reaction may be effected by maintaining the ester at a pH of 7.5 to 9.5 until hydrolysis is complete. Most suitably a readily hydrolysable ester such as the methoxymethyl ester is employed in this process. The pH may be maintained in the desired range in a pH-stat by the addition of a solution of a base such as LiOH, NaOH, KOH, $NaHCO_3$ or the like at a rate that prevents accumulation of excess base which would cause the pH to increase

unacceptably, or by the use of a suspension of finely divided $Ca(OH)_2$, $Mg(OH)_2$, MgO, $MgCO_3$ or the like. Other bases which may be employed for hydrolysis include $Ba(OH)_2$, $Sr(OH)_2$ and the like.

Suitable methods of hydrogenolysis of esters of compounds (I) include hydrogenation in the presence of a transition metal catalyst. Suitable hydrogenolysable esters are benzyl and substituted benzyl esters discussed above. The pressure of hydrogen used in the reaction may be low, medium or high, but in general an approximately atmospheric or slightly super-atmospheric pressure of hydrogen is preferred. The transition metal catalyst employed is preferably palladium on charcoal. The hydrogenation may be effected in any inert solvent in which the ester is soluble such as tetrahydrofuran or the like. If this hydrogenation is carried out in the presence of a base then a salt of compounds (I) is produced. Suitable bases for inclusion include $NaHCO_3$, $KHCO_3$, $Na_2CO_3$, $K_2CO_3$, $CaCO_3$, $MgCO_3$, $LiHCO_3$, $NH_4OCOCH_3$, $Mg(OCOCH_3)_2$, $Mg(OCOH)_2$ and the like. If no base is present then hydrogenation leads to the preparation of an acid within formula (I) which may then be neutralised if desired to yield a salt. Suitable bases which may be used to neutralise acids within formula (I) include LiOH, NaOH, $NaHCO_3$, KOH, $Ca(OH)_2$, $Ba(OH)_2$, MgO, $Mg(OH)_2$, $NH_4OH$ and $N(C_2H_5)_3$.

The lithium salts of compounds (I) tend to be more easily prepared in pure crystalline form than other salts of compounds (I). It is therefore often convenient

to first form the lithium salt and then convert this into a further salt by ion-exchange, for example by passing a solution of the lithium salt through a bed of a cation exchange resin in sodium, potassium, calcium, ammonium or like form. Suitable cation exchange resins include Amberlite IR 120 and equivalent resins.

The salts of acids (I) may be converted to esters in conventional manner, for example by reaction with a reactive halide in solution in dimethylformamide or like solvent. Esters may similarly be prepared by the reaction in an inert solvent of an acid (I) with an alcohol in the presence of a condensation promoting agent such as dicyclohexylcarbodiimide.

In general salts of the compounds (I) above are prepared from the free acid by known methods as disclosed in British Patent Specification Number: 1508977 for the preparation of clavulanic acid salts. Similarly, esters of compounds (I) are prepared from the free acid by known methods as disclosed in British Patent Specification Number: 1508978 for the preparation of clavulanic acid esters.

The following Examples illustrate the invention.

EXAMPLE 1

The preparation of benzyl 9-pyrr-2-yldeoxyclavulanate

Benzyl clavulanate (2.9 g, 10 mmol) was dissolved in anhydrous dichloromethane (50 ml) containing pyrrole (10 ml), at -10°C under an atmosphere of nitrogen. To this stirred solution was added boron trifluoride etherate (0.5 ml, 4 mmol), and the reaction was monitored by t.l.c. An additional aliquot of boron trifluoride etherate (1.0 ml, 8 mmol) was added after three hours; the reaction temperature being maintained all the while at -10°C - 0°C. The reaction mixture was worked-up after six hours despite the presence of unreacted benzylclavulanate (as shown by t.l.c.) due to the gradually darkening colouration of the reaction solution. The reaction mixture was diluted with chloroform (100 ml), washed with aqueous sodium bicarbonate (150 ml), dried ($MgSO_4$), and evaporated *in vacuo* to afford a foam (4.5 g). This was subjected to column chromatography on silica gel (60 g) using ethylacetate : cyclohexane (1:4). The appropriate fractions were collected, combined, and evaporated *in vacuo* to afford benzyl 9-pyrr-2-yldeoxyclavulanate (0.35 g) as an oil, i.r. (liq film) 3380, 1790, 1740, 1695 $cm^{-1}$, n.m.r. ($CDCl_3$) 3.00 (1H, d, *J* = 17 Hz, 6β-*H*), 3.36 (2H, d, *J* = 8 Hz, 9-$C\underline{H}_2$), 3.45 (1H, dd J= 17 and 3 Hz, 6α-*H*), 4.78 (1H, t, *J* = 8 Hz, 8-*H*), 5.05 (1H, s, 3-*H*), 5.17 (2H, s, $C\underline{H}_2Ph$), 5.66 (1H, d, *J* = 3 Hz, 5-H), 5.82 (1H, m, *Ha*), 6.06 (1H, m, *Hb*), 6.60 (1H, m, *Hc*), 7.30 (5H, s, *Ph*), 7.8 (1H, br, N-*Hd*) (disappeared on $D_2O$ shake) p.p.m.

EXAMPLE 2

The preparation of lithium 9-pyrr-2-yldeoxyclavulanate

Benzyl 9-pyrr-2-yldeoxyclavulanate (0.3 g) was dissolved in tetrahydrofuran (15 ml) containing water (10 drops), and was hydrogenated over 10% Palladium on charcoal (0.12 g) for 2 hours. The catalyst was filtered off, and washed with tetrahydrofuran and water. The combined filtrate and washings were taken to pH 7 with aqueous lithium hydroxide. The solvents were removed *in vacuo* to yield a gum (0.27 g). This was triturated with acetone : ether (1:1) (20 ml) to afford an impure brown solid (0.12 g). This was subjected to column chromatography on silica gel (15 g) using n-butanol : ethanol : water (4:1:1) as eluent. The appropriate fractions were collected, combined, and evaporated *in vacuo* to yield lithium 9-pyrr-2-yldeoxyclavulanate (0.06 g) as a solid, i.r. (KBr) 3380, 1772, 1690, 1604 $cm^{-1}$, n.m.r. ($D_2O$) 3.06 (1H, d *J* = 17 Hz, 6β-*H*), 3.40 (2H, d *J* = 8 Hz, 9-$CH_2$), 3.54 (1H, dd *J* = 17 and 3 Hz, 6α-*H*), 8-H (obscured by HOD), 4.89 (1H, s, 3-*H*), 5.68 (1H, d *J* = 3 Hz, 5-*H*), 5.89 (1H, m, $H_a$), 6.07 (1H, m, $H_b$), 6.74 (1H, m, $H_c$) p.p.m.

EXAMPLE 3

The preparation of benzyl 9-(1-methyl)pyrr-2-yldeoxyclavulanate.

Benzyl clavulanate (1.4g) was dissolved in anhydrous dichloromethane (50ml) containing 1-methylpyrrole (5ml), at -30°C under an atmosphere of nitrogen. To this stirred solution was added boron trifluoride etherate (0.1ml) and the reaction was followed by t.l.c. After two hours additional boron trifluoride etherate (0.15ml) was added; the reaction temperature being maintained all the while at -30° - -20°C. The reaction was worked up after four hours despite the presence of unreacted benzyl clavulanate (as shown by t.l.c), due to the gradually darkening reddish colour of the reaction solution. The reaction mixture was diluted with chloroform (100ml), washed with saturated sodium bicarbonate (150ml), dried ($MgSO_4$), and evaporated *in vacuo* to a small volume. Toluene (100ml) was added and the solution evaporated *in vacuo* to a gum (1.3g). This was subjected to column chromatography on silica gel (60g) using ethyl acetate : cyclohexane (1:4). The appropriate fractions were collected, combined, and evaporated *in vacuo* to afford benzyl 9-(1-methyl)pyrr-2-yldeoxyclavulanate (0.24g) as an oil, i.r. (liq film) 1800, 1750, 1699 $cm^{-1}$, n.m.r ($CDCl_3$) 2.98 (1H, d, *J* = 17Hz; 6β-*H*), 3.33 (2H, d, *J* = 7Hz, 9-$CH_2$), 3.40 (3H, s, N-*Me*), 3.43 (1H, d, *J* = 3Hz, 6α-*H*), 4.72 (1H, dt, *J* = 7 and 1Hz, 8-*H*), 5.05 (1H, d, *J* = 1Hz, 3-H), 5.15 (2H, s, $CH_2$Ph), 5.65 (1H, d, *J* = 3Hz, 5 -H), 5.78 (1H, m, $H_a$), 5.97 (1H, dd, *J* 3 and 2Hz, $H_b$), 6.48 (1H, dd, *J* 2 and 2Hz, $H_c$), 7.30 (5H, s, *Ph*) p.p.m. The n.m.r. spectrum indicated the presence of a minor isomer (approx. 15%); this was adjudged to be benzyl 9-(1-methyl)-pyrr-3-yldeoxyclavulanate, n.m.r. ($CDCl_3$) in part 3.34 (d, *J* = 7Hz, 9-$CH_2$), 3.52 (s, N-*Me*), 4.80 (dt, *J* = 7 and 1 Hz, 8-*H*), 5.02 (d, *J* = 1Hz, 3-*H*), 5.89 and 6.29 (2m, pyrrole protons) p.p.m.

EXAMPLE 4

The preparation of lithium 9-(1-methyl)pyrr-2-yldeoxy-clavulanate.

The mixture of isomers from Example 3 (0.2g) was dissolved in tetrahydrofuran (20ml) containing water (6 drops), and hydrogenated over 10% palladium on charcoal (0.07 g) for two hours. The catalyst was filtered off, and washed with tetrahydrofuran and water. The combined washings and filtrate were taken to pH 7.5 with aqueous lithium hydroxide. The solvents were then evaporated *in vacuo* (n-propanol added to facilitate evaporation) to afford a creamy-white solid (0.11g). This was triturated with acetone :ether (1:3) to afford lithium 9-(1-methyl)pyrr-2-yldeoxyclavulanate (0.08 g) as a creamy-white solid, i.r. (KBr) 3400, 1770 sh, 1760, 1700, 1620 sh, 1608 $cm^{-1}$, n.m.r. ($D_2O$) 3.05 (1H, d, $J$ = 17Hz, 6β-$H$), 3.35 (2H, d, $J$ = 8Hz, 9-$CH_2$), 3.50 (3H, s, N-Me), 3.55 (1H, dd, $J$ = 17 and 3Hz, 6α-$H$), (8-H obscured by HOD), 4.88 (1H, s, 3-$H$), 5.70 (1H, d, $J$ = 3Hz, 5 -$H$), 5.84 - 6.05 and 6.64 (3H, 3m, pyrrole protons) p.p.m. A small amount (approx. 15%) of lithium 9-(1-methyl)pyrr-3-yldeoxyclavulanate was detectable by i.r. and n.m.r.

EXAMPLE 5

The preparation of benzyl 9-(5-methyl)fur-2-yldeoxyclavulanate

Benzyl clavulanate (2.9 g, 10 mmol) was dissolved in anhydrous dichloromethane (50 ml) containing 2-methylfuran (20 ml), at -30°C under an atmosphere of nitrogen. To this stirred solution was added boron trifluoride etherate (0.5 ml, 4 mmol), and the reaction was monitored by t.l.c.; the temperature being maintained at -30°C - -10°C. When three hours had elapsed the reaction mixture was diluted with dichloromethane (100 ml), washed with aqueous sodium bicarbonate (150 ml), dried ($MgSO_4$), and evaporated in vacuo to afford a foam (4.0 g). This foam was subjected to column chromatography on silica gel (60 g) using ethylacetate : cyclohexane (1:4). The appropriate fractions were collected, combined, and evaporated in vacuo to afford benzyl 9-(5-methyl)fur-2-yldeoxyclavulanate (0.65 g) as an oil, i.r. (liq film) 1805, 1755, 1700, 1620 $cm^{-1}$, n.m.r. ($CDCl_3$) 2.22 (3H, s, $CH_3$), 3.02 (1H, d $J$ = 17 Hz, 6β-$H$), 3.36 (2H, d $J$ = 7 Hz, 9-$CH_2$), 3.45 (1H, dd J = 17 and 3 Hz, 6α-$H$), 4.79 (1H, dt $J$ = 7 and 1 Hz, 8-$H$), 5.08 (1H, br s, 3-$H$), 5.19 (2H, s, $CH_2Ph$), 5.67 (1H, d $J$ = 3 Hz, 5-H), 5.75 - 5.95 (2H, m, furan protons), 7.31 (5H, s, Ph) p.p.m. A more polar β-lactam containing impurity was subsequently eluted and collected.

EXAMPLE 6

Preparation of lithium 9-(5-methyl)fur-2-yldeoxyclavulanate

Benzyl 9-(5-methyl)fur-2-yldeoxyclavulanate (0.5 g) was dissolved in tetrahydrofuran (30 ml) containing water (6 drops), and was hydrogenated over 10% Palladium on charcoal (0.2 g) for ninety minutes. The catalyst was filtered off, and washed with tetrahydrofuran and water. The combined washings and filtrate were taken to pH 7.2 with aqueous lithium hydroxide. The solvents were removed *in vacuo* to yield a brownish gum. This was washed with ethylacetate (10 ml) and then triturated with acetone : ether (1:3) (20 ml) to afford a gummy solid (0.12 g). This was triturated with acetone : ether (1:2) to afford lithium 9-(5-methyl)fur-2-yldeoxyclavulanate (0.06 g) as an off-white solid, i.r. (KBr) 3430, 1764, 1701, 1610 $cm^{-1}$, n.m.r. ($D_2O$) 2.18 (3H, s, $CH_3$), 3.00 (1H, d *J* = 17 Hz, 6β-*H*), 3.34 (2H, d *J* = 8 Hz, 9-$CH_2$), 3.50 (1H, dd *J* = 17 and 3 Hz, 6α-H), 4.81 (1H, t *J* = 8 Hz, 8-*H*), 4.89 (1H, s, 3-*H*), 5.65 (1H, d *J* = 3 Hz, 5-*H*), 5.90 (2H, br s, furan protons) p.p.m.

EXAMPLE 7

Preparation of benzyl 9-(3-methyl)thien-2-yldeoxyclavulanate

Benzyl clavulanate (2.9 g, 10 mmol) was dissolved in a solvent mixture of anhydrous dichloromethane (25 ml) and 3-methylthiophen (25 ml), at -50°C under an atmosphere of nitrogen. To this stirred solution was added boron trifluoride etherate (0.1 ml, 1 mmol), and the reaction was monitored by t.l.c. A further amount of boron trifluoride etherate (0.15 ml, 1.5 mmol) was added after three hours. The reaction temperature was maintained at -40°C - -20°C for 20 hours. The reaction mixture was then diluted with chloroform (200 ml), washed with aqueous sodium bicarbonate (200 ml), dried ($MgSO_4$), and evaporated in vacuo to afford a gum (2.0 g). This was subjected to column chromatography on silica gel (50 g) using ethylacetate : cyclohexane (1:3). The title compound was eluted first with a few impurities. The fractions containing the title compound were evaporated in vacuo, and rechromatographed on silica gel (60 g) using ethylacetate : cyclohexane (1:4). The appropriate fractions were collected, combined, and evaporated in vacuo to yield benzyl 9-(3-methyl)thien-2-yldeoxyclavulanate (0.55 g) as an oil, i.r. (liq film) 1805, 1750, 1700 $cm^{-1}$, n.m.r. ($CDCl_3$) 2.10 (3H, s, $CH_3$), 3.03 (1H, d J = 17 Hz, 6β-H), 3.46 (1H, dd J = 17 and 3 Hz, 6α-H), 3.48 (2H, d J = 9 Hz, 9-$CH_2$), 4.78 (1H, dt J = 9 and 1 Hz, 8-H), 5.06 (1H, s, 3-H, 5.18 (2H, s, $CH_2Ph$), 5.69 (1H, d J = 3 Hz, 5-H), 6.74 (1H, d J = 5.5 Hz, 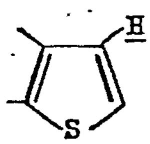 ), 6.97 (1H, d J = 5.5 Hz, 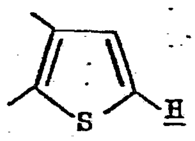 ), 7.30 (5H, s, Ph) p.p.m.

EXAMPLE 8

Preparation of p-nitrobenzyl 9-(3-methyl)thien-2-yldeoxyclavulanate

p-Nitrobenzyl clavulanate (3.34 g, 10 mmol) was dissolved in a solvent mixture of anhydrous dichloromethane (30 ml) and 3-methylthiophene (20 ml), at 0°C under an atmosphere of nitrogen. To this stirred solution was added boron trifluoride etherate (0.4 ml, 3 mmol), and the reaction was monitored by t.l.c. A further amount of boron trifluoride etherate (0.4 ml, 3 mmol) was added after 3 hours. The reaction temperature was maintained at -20° - 0°C for 6 hours; and then it was diluted with chloroform (100 ml) (there was a considerable amount of insoluble material), washed with aqueous sodium bicarbonate (200 ml), dried ($MgSO_4$), and evaporated in vacuo to afford a gum (1.1 g). This was subjected to column chromatography on silica gel (65 g) using ethylacetate : cyclohexane (1:4). The appropriate fractions were collected, combined, and evaporated in vacuo to yield p-nitrobenzyl 9-(3-methyl)-thien-2-yldeoxyclavulanate (0.38 g) as an oil, i.r (liq film) 2990, 1805, 1755, 1695, 1610 $cm^{-1}$, n.m.r. ($CDCl_3$) 2.10 (3H, s, $C\underline{H}_3$), 3.07 (1H, d $\underline{J}$ = 17 Hz, 6β-$\underline{H}$), 3.48 (2H, d $\underline{J}$ = 8 Hz, 9-$C\underline{H}_2$), 3.50 (1H, dd $\underline{J}$ = 17 and 3 Hz, 6α-$\underline{H}$), 4.76 (1H, dt $\underline{J}$ = 8 and 1 Hz, 8-$\underline{H}$), 5.10 (1H, br s, 3-$\underline{H}$), 5.26 (2H, ABq $\underline{J}$ ≃ 14 Hz, $C\underline{H}_2$-Ar), 5.70 (1H, d $\underline{J}$ = 3 Hz, 5-$\underline{H}$), 6.76 and 6.98 (2H, 2d $\underline{J}$ ≃ 5.5 Hz, thienyl protons), 7.41 and 8.14 (4H, 2d $\underline{J}$ = 8 Hz, aromatic protons) p.p.m.

EXAMPLE 9

Preparation of lithium 9-(3-methyl)thien-2-yldeoxyclavulanate

p-Nitrobenzyl 9-(3-methyl)thienyldeoxyclavulanate (0.34 g) was dissolved in tetrahydrofuran (10 ml) containing water (5 drops), and was hydrogenated over prehydrogenated 10% Palladium on charcoal (0.35 g) for 75 minutes. The catalyst was filtered off, and washed with tetrahydrofuran and water. The combined washings and filtrate were taken to pH 7.2 with aqueous lithium hydroxide. The solvents were removed *in vacuo* to yield a brownish gum. This was triturated with acetone (5 ml) to yield a solid (0.075 g). The mother liquor was evaporated *in vacuo*, and triturated with acetone:ether (1:2) to afford lithium 9-(3-methyl)thienyldeoxyclavulanate (0.05 g) as a solid, i.r. (KBr) 3390, 1765, 1696, 1610 $cm^{-1}$, n.m.r. ($D_2O$) 2.10 (3H, s, $CH_3$), 2.9-3.7 (4H, m, 6β-H, 6α-H and 9-$CH_2$), 8-H (obscured by HOD), 4.88 (1H, s, 3-H), 5.67 (1H, brs, 5-H), 6.83 and 7.12 (2H, 2m, thienyl protons) ppm.

EXAMPLE 10

The preparation of benzyl 9-indol-3-yldeoxyclavulanate

Benzyl clavulanate (2.9 g, 10 mmol) and indole (2.7 g, 23 mmol) were dissolved in anhydrous dichloromethane (50 ml) at -10°C, under an atmosphere of nitrogen. To this stirred solution was added boron trifluoride etherate (0.5 ml, 4 mmol), and the reaction was monitored by t.l.c. Additional aliquots of boron trifluoride etherate were added after 3 hours (0.5 ml) and 6 hours (0.5 ml). The reaction temperature was maintained at -20°C - 0°C for 7½ hours; it was then diluted with chloroform (100 ml), washed with aqueous sodium bicarbonate (150 ml), washed with brine (150 ml), dried ($MgSO_4$), and evaporated in vacuo to afford a foam (6.0 g). This was subjected to column chromatography on silica gel (65 g) using ethylacetate : cyclohexane (1:5). The first component to be eluted was a non-β-lactam containing impurity. Subsequently the title compound was eluted; fractions containing this were combined and evaporated in vacuo to afford benzyl 9-indol-3-yldeoxyclavulanate (0.33 g) as an oil, i.r. (liq film) 3420, 1800, 1745, 1699 $cm^{-1}$, n.m.r. ($CDCl_3$) 3.03 (1H, d J = 17 Hz, 6β-H), 3.45 (1H, dd J = 17 and 3 Hz, 6α-H), 3.51 (2H, dd J 8 and 0.5 Hz, 9-$CH_2$), 4.86 (1H, dt J = 8 and 1 Hz, 8-H), 5.05 (1H, d J = 1 Hz, 3-H), 5.11 (2H, s, $CH_2$Ph), 5.69 (1H, d J = 3 Hz, 5-H), 6.79 (1H, m on $D_2O$ shake goes to d J ≃ 0.5 Hz, CH-NH), 7.0-7.6 (9H,m, aromatic protons), 7.9 (1H, br s, NH) (disappeared on $D_2O$ shake) p.p.m.

EXAMPLE 11

The preparation of lithium 9-indol-3-yldeoxyclavulanate

Benzyl 9-indol-3-yldeoxyclavulanate (0.3 g) was dissolved in tetrahydrofuran (15 ml) containing water (5 drops), and was hydrogenated over 10% Palladium on charcoal (0.1 g) for 2 hours. The catalyst was filtered off, and washed with tetrahydrofuran and water. The combined washings and filtrate were taken to pH 7.2 with aqueous lithium hydroxide. The solvents were then concentrated *in vacuo* to 50 ml volume; and this was then washed with ethyl acetate (2 x 40 ml). The aqueous layer was evaporated *in vacuo* to afford a gum (0.2 g). This was then triturated with acetone : ether (1:1) (4 ml) to yield lithium 9-indol-3-yldeoxyclavulanate (0.17 g) as a solid, i.r. (KBr) 3380, 1765, 1695, 1610 $cm^{-1}$, n.m.r. ($D_2O$) 3.00 (1H, d *J* = 17 Hz, 6β-*H*), 3.4-3.7 (3H, m, 6α-*H* and 9-$CH_2$), 8-*H* (obscured by HOD), 4.85 (1H, s, 3-*H*), 5.68 (1H, d *J* ≏ 3 Hz), 7.1-7.7 (5H, m, indolyl protons) p.p.m.

EXAMPLE 12

Preparation of benzyl 9-(3-methyl)benzofur-2-yldeoxyclavulanate

Benzyl clavulanate (4.7 g) and 3-methylbenzofuran (3.7 g) were dissolved in anhydrous dichloromethane (50 ml) at -20°C, under an atmosphere of nitrogen. To this stirred solution was added boron trifluoride etherate (0.5 ml). The reaction was monitored by t.l.c., and the temperature was maintained below -20°C. The reaction was worked-up after 90 minutes, despite the presence of approx. 50% unreacted benzyl clavulanate, due to a considerable darkening in the colouration of the reaction mixture. The reaction mixture was diluted with dichloromethane (200 ml), washed with aqueous sodium bicarbonate (150 ml) containing brine (50 ml), washed with water (100 ml), dried ($MgSO_4$), and evaporated in vacuo to afford a mobile oil (6.5 g). This was subjected to column chromatography on silica gel (70 g) using ethyl acetate : cyclohexane

(2:11) as eluent. The appropriate fractions were collected, combined, and evaporated in vacuo to afford as an oil, benzyl 9-(3-methyl)benzofur-2-yldeoxyclavulanate (0.20 g), i.r. (liq film) 1805, 1755, 1700, 1635, 1615 $cm^{-1}$, n.m.r. ($CDCl_3$) 2.10 (3H, s, $CH_3$), 3.03 (1H, d, J = 17 Hz, 6β-H), 3.46 (1H, dd, J = 17 and 3 Hz, 6α-H), 3.50 (2H, d, J = 8 Hz, 9-$CH_2$), 4.81 (1H, dt J = 8 and 1 Hz, 8-H), 5.05 (1H, d, J = 1 Hz, 3-H), 5.14 (2H, ABq, $CH_2Ph$), 5.69 (1H, d, J = 3 Hz, 5-H), 7.1 - 7.5 (9H, m, aromatic) p.p.m.

EXAMPLE 13

Preparation of p-nitrobenzyl 9-benzothienyldeoxyclavulanate

p-Nitrobenzylclavulanate (3.4 g, 10 mmol) was dissolved in anhydrous dichloromethane (80 ml) containing benzothiophen (6 ml). To this stirred solution at 0°C, under an atmosphere of nitrogen, was added boron trifluoride etherate (0.55 ml, 5 mmol). The reaction was monitored by t.l.c., and the temperature was maintained below 0°C. After 150 minutes the reaction mixture was diluted with dichloromethane (300 ml), washed with aqueous sodium bicarbonate (300 ml), dried ($MgSO_4$), and evaporated *in vacuo* to afford a yellow gum (8.0 g). This was subjected to column chromatography on silica gel (65 g) using ethyl acetate : cyclohexane (1:6)→(1:5)→(1:4) as eluent. The appropriate fractions were collected, combined, and evaporated *in vacuo* to afford as an oil, p-nitrobenzyl 9-benzothienyldeoxyclavulanate (0.074 g) i.r. (liq film) 1800, 1755, 1610 $cm^{-1}$, n.m.r. ($CDCl_3$) 3.07 (1H, d, *J* = 17 Hz, 6β-*H*), 3.50 (1H, dd, *J* = 17 and 3 Hz, 6α-*H*), 3.60 (2H, d, *J* 8 Hz, 9-$CH_2$), 4.82 (1H, dt, *J* = 8 and 1 Hz, 8-*H*), 5.11 (1H,

s, 3-H), 5.16 (2H, ABq J = 12 Hz, $COOCH_2$), 5.73 (1H, d, J = 3 Hz, 5-H), and 6.9 - 8.1 (9H, m, aromatic protons) p.p.m.

EXAMPLE 14

Preparation of methyl 9-benzothienyldeoxyclavulanate

Methyl clavulanate (2.13 g, 10 mmol) was dissolved in anhydrous dichloromethane (60 ml) containing benzothiophen (8 ml), at -10°C under an atmosphere of nitrogen. To this stirred solution was added boron trifluoride etherate (0.6 ml). After two hours a further amount of boron trifluoride etherate (0.6 ml) was added. The reaction was monitored by t.l.c., and the temperature maintained below 0°C. After four hours the reaction mixture was diluted with dichloromethane (300 ml), washed with aqueous sodium bicarbonate (200 ml) containing brine (100 ml), washed with water (100 ml), dried ($MgSO_4$), and evaporated in vacuo to afford a gum (7.0 g). This gum was subjected to column chromatography on silica gel (65 g) using ethyl acetate : cyclohexane (2:9) as eluent. The appropriate fractions were collected, combined, and evaporated in vacuo to afford as an oil, methyl 9-benzothienyldeoxyclavulanate (0.02 g), i.r. (liq film) 1805, 1765, 1700, 1610 $cm^{-1}$, n.m.r. ($CDCl_3$) 3.05 (1H, d, $J$ = 18 Hz, 6β-H), 3.51 (1H, dd, $J$ = 18 and 3 Hz, 6α-H), 3.65 (2H, d, $J$ = 9 Hz, 9-$CH_2$), 3.75 (3H, s, $COOCH_3$), 4.89

(1H, dt, J = 9 and 1 Hz, 8-H), 5.06 (1H, d, J = 1 Hz, 3-H), 5.73 (1H, d, J = 3 Hz, 5-H), 6.7 - 7.9 (5H, m, aromatic protons) p.p.m.

Demonstration of Effectiveness

In a standard MIC synergy test the following results were obtained.

| | Concentration of Inhibitor | Staph.aureus Russell | Kleb. aerogenes E70 | E.coli JT39 |
|---|---|---|---|---|
| Ampicillin alone | | 500 | 500 | 2000 |
| Ampicillin + Compound of Example 4 | 5.0 μg/ml<br>1.0 μg/ml | -<br>0.15 | 12.5<br>25.0 | 16<br>500 |
| Ampicillin + Compound of Example 6 | 5.0 μg/ml<br>1.0 μg/ml | -<br>0.15 | 3.1<br>12.5 | 4.0<br>31.2 |
| Compound of Example 4 alone | | 8.0 | 250 | 125 |
| Compound of Example 6 alone | | 2.0 | 500 | 125 |

WHAT WE CLAIM IS:

1. A compound of the formula (I):

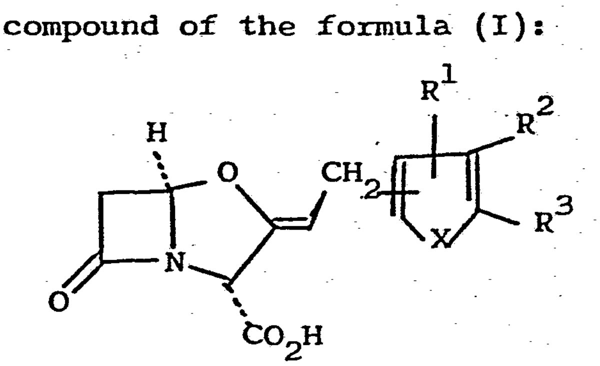

(I)

and pharmaceutically acceptable salts and in-vivo hydrolysable esters thereof wherein $R^1$ is a hydrogen atom or a $C_{1-6}$ alkyl group, $R^2$ is a hydrogen atom or a $C_{1-6}$ alkyl group, $R^3$ is a hydrogen atom or a $C_{1-6}$ alkyl group, or $R^2$ and $R^3$ together with the carbon atoms to which they are attached form a benzene ring which optionally is substituted by a $C_{1-6}$ alkyl, $C_{1-6}$ alkoxy or hydroxy group; and X is a sulphur or oxygen atom or is a group $NR^4$ wherein $R^4$ is a hydrogen atom or a $C_{1-6}$ alkyl group.

2. A compound as claimed in claim 1 wherein $R^2$ and $R^3$ together with the carbon atoms to which they are attached represent an unsubstituted benzene ring.

3. A compound as claimed in claim 1 wherein $R^1$, $R^2$ and $R^3$ are independently selected from the group consisting of hydrogen and methyl.

4. A compound as claimed in claim 1 of the formula (II):

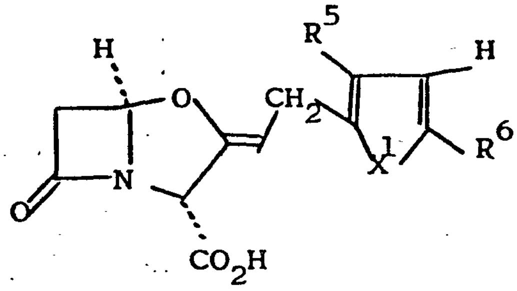

(II)

and pharmaceutically acceptable salts and in-vivo hydrolysable esters thereof wherein $X^1$ is an oxygen or sulphur atom or a -NH- or $-N(CH_3)-$ group; $R^5$ is a hydrogen atom or a methyl group and $R^6$ is a hydrogen atom or methyl group.

5. A compound as claimed in claim 1 selected from the group consisting of:

9-pyrr-2-yldeoxyclavulanic acid,
9-(1-methyl)pyrr-2-yldeoxyclavulanic acid,
9-(3-methyl)thien-2-yldeoxyclavulanic acid,
9-(5-methyl)fur-2-yldeoxyclavulanic acid,
9-(3-methyl)benzofur-2-yldeoxyclavulanic acid,
9-indol-3-yldeoxyclavulanic acid,
9-thionaphthyldeoxyclavulanic acid,

and pharmaceutically acceptable salts thereof.

6. A compound as claimed in any of claims 1 to 5 in the form of a sodium or potassium salt.

7. A pharmaceutical composition which comprises a compound of the formula (I) or a pharmaceutically acceptable salt or in-vivo hydrolysable ester thereof and a pharmaceutically acceptable carrier.

8. A composition as claimed in claim 7 which also comprises a penicillin or cephalosporin.

9. A process for the preparation of a compound of the formula (I) or a pharmaceutically acceptable salt or in-vivo hydrolysable ester thereof which process comprises the reaction of an ester of clavulanic acid of the formula (III):

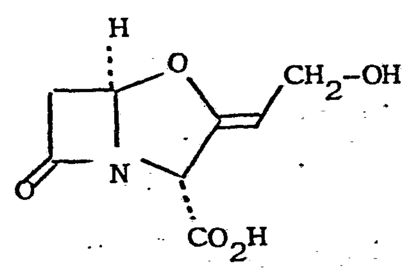

(III)

with a compound of the formula (IV):

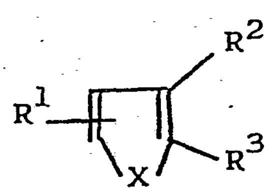

(IV)

wherein X, $R^1$, $R^2$ and $R^3$ are as defined in claim 1; in the presence of a catalyst; and thereafter:

i) converting any ester that is not in-vivo

hydrolysable into a free acid or a pharmaceutically acceptable salt or in-vivo hydrolysable ester;

ii) optionally converting any in-vivo hydrolysable ester into a free acid, a pharmaceutically acceptable salt or different in-vivo hydrolysable ester.

10. A process as claimed in claim 9 wherein the catalyst is boron trifluoride etherate.

CLAIMS FOR AUSTRIA

WHAT WE CLAIM IS:

1. A process for the preparation of a compound of the formula (I):

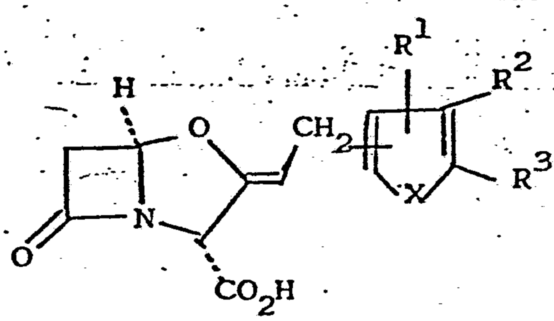

(I)

and pharmaceutically acceptable salts and in-vivo hydrolysable esters thereof wherein $R^1$ is a hydrogen atom or a $C_{1-6}$ alkyl group, $R^2$ is a hydrogen atom or a $C_{1-6}$ alkyl group, $R^3$ is a hydrogen atom or a $C_{1-6}$ alkyl group, or $R^2$ and $R^3$ together with the carbon atoms to which they are attached form a benzene ring which optionally is substituted by a $C_{1-6}$ alkyl, $C_{1-6}$ alkoxy or hydroxy group; and X is a sulphur or oxygen atom or is a group $NR^4$ wherein $R^4$ is a hydrogen atom or a $C_{1-6}$ alkyl group; which process comprises the reaction of an ester of clavulanic acid of the formula (III):

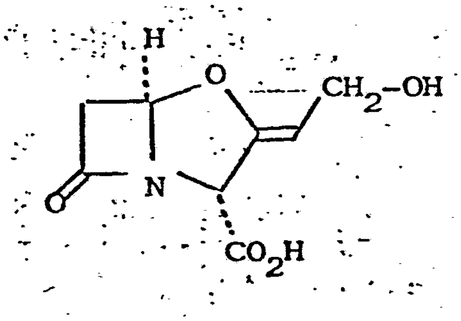

(III)

with a compound of the formula (IV):

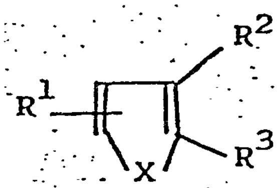

(IV)

wherein X, $R^1$, $R^2$ and $R^3$ are as defined in claim 1; in the presence of a catalyst; and thereafter:

i) converting any ester that is not in-vivo hydrolysable into a free acid or a pharmaceutically acceptable salt or in-vivo hydrolysable ester;

ii) optionally converting any in-vivo hydrolysable ester into a free acid, a pharmaceutically acceptable salt or different in-vivo hydrolysable ester.

2. A process as claimed in claim 1 wherein the catalyst is boron trifluoride etherate.

3. A process as claimed in either claim 1 or claim 2 wherein clavulanic acid is in the form of hydrolysable ester.

4. A process as claimed in either claim 1 or claim 2 wherein clavulanic acid is in the form of a hydrogenolysable

ester.

5. A process for the preparation of a pharmaceutical composition which comprises bringing into association a compound of the formula (I) as defined in claim 1 with a pharmaceutically acceptable carrier.

6. A process as claimed in claim 5 which also comprises a penicillin or cephalosporin.

0025287

European Patent Office

# EUROPEAN SEARCH REPORT

Application number
EP 80302837.2

| Category | Citation of document with indication, where appropriate, of relevant passages | Relevant to claim | CLASSIFICATION OF THE APPLICATION (Int. Cl. 3) |
|---|---|---|---|
| | DE - A1 - 2 747 350 (GLAXO)<br>+ Pages 1-17 +<br>-- | 1,7-9 | C 07 D 498/04<br>A 61 K 31/42//<br>(C 07 D 498/04<br>C 07 D 205/00<br>C 07 D 263/00) |
| A | DE - A1 - 2 704 104 (BEECHAM)<br>+ Pages 1-9,126,164,165 +<br>-- | 1,7,8 | |
| A | GB - A - 2 006 769 (GLAXO)<br>+ Totality +<br>---- | | |

TECHNICAL FIELDS SEARCHED (Int.Cl. 3)

C 07 D 498/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 17-10-1980 | JANISCH |

EPO Form 1503.1 06.78